# FASCICULE DE BREVET EUROPEEN

(11) **EP 0 638 307 B1**
(45) Date de publication et mention de la délivrance du brevet: **26.11.1997**
(21) Numéro de dépôt: 94401840.7
(22) Date de dépôt: 10.08.1994
(51) Int. Cl.: A61K 7/16

(54) **Composition de dentifrice anhydre**
Wasserfreie Zahnpasta
Anhydrous dentifrice

(30) Priorité: 11.08.1993 FR 9309867
(43) Date de publication de la demande: 15.02.1995
(73) Titulaire: L'OREAL, 75008 Paris (FR)
(72) Inventeur: Ascione, Jean-Marc, F-75018 Paris (FR)
(74) Mandataire: Casalonga, Axel

(56) Documents cités:
- EP-A- 0 138 615
- WO-A-86/02830
- DE-A- 2 033 678
- FR-A- 2 516 792
- US-A- 4 071 615

## Description

La présente invention est relative à de nouvelles compositions de dentifrice anhydre à base de glycérine, d'hydroxyéthylcellulose à chaîne hydrophobe et de silice pyrogénée ainsi qu'à leur utilisation pour l'hygiène bucco-dentaire.

Les dentifrices sont bien connus dans l'état de la technique et doivent réunir de nombreuses qualités tant sur le plan de l'aspect tel que l'homogénéité, de la rhéologie, de la conservation et des pouvoirs moussants, des propriétés de nettoyage et de polissage ....

On cherche en particulier des compositions présentant lors de leur utilisation un aspect lisse, homogène, brillant, ayant une viscosité constante, une consistance appropriée pour former un ruban, adhérant à la brosse à dents sans toutefois s'étaler de trop. Ces compositions doivent par ailleurs présenter un pouvoir de nettoyage et de polissage élevé, pour conférer un éclat à l'émail tout en présentant une abrasivité faible à l'égard de la dentine.

Les compositions de dentifrices de l'état de la technique sont ]e plus souvent sous forme aqueuse, ce qui peut poser des problèmes quant à l'utilisation dans de tels dentifrices, d'agents actifs peu ou pas stables en milieu aqueux tels que des enzymes, des agents de blanchiment, des libérateurs d'oxygène, des vitamines etc... Les compositions nécessitent souvent l'utilisation d'agents conservateurs.

On connaît par le brevet US-A 4 071 615 des compositions de dentifrice anhydre à base de glycérine, d'agents épaississants tels que l'hydroxyéthylcellulose et la carboxyméthylcellullose, et de silice.

Ces compositions ont l'inconvénient d'avoir une consistance insuffisante, les compositions préparées à l'aide des agents épaississants envisagés présentent un caractère filant difficilement acceptable pour un dentifrice.

Le brevet EP-A 138 615, prévoit l'utilisation de silice épaississante ayant une granulométrie de 2 à 20 µm.

Les compositions décrites dans ce document mettant en oeuvre les silices précipitées ou pyrogénées présentant une granulométrie de 2 à 20 µm, ont l'inconvénient de conduire en milieu anhydre à des compositions de viscosité insuffisante et peu stables au stockage.

Le document WO-A-8602830 décrit une composition de dentifrice anhydre dont l'agent gélifiant peut être choisi parmi, entre autres, l'hydroxyethylcellulose, les silices et leurs mélanges. Les agents gélifiants recommandés comprennent l'hydroxyéthylcellulose, les aérogels de silice et leurs mélanges.

La demanderesse a mis au point une nouvelle composition de dentifrice anhydre permettant l'introduction d'actifs peu ou pas stables en milieu aqueux et présentant à l'utilisation les caractères lisse, homogène, brillant, une viscosité, une consistance, un pouvoir nettoyant et de polissage mentionnés ci-dessus. La composition a par ailleurs, l'avantage d'être stable au stockage. Selon l'invention et tout au long de la description qui va suivre, on entend par composition anhydre, une composition contenant moins de 3 % en poids d'eau. Cette très faible proportion en eau étant dûe aux traces d'eau présentes dans certaines matières premières utilisées conformément à l'invention.

La demanderesse a découvert plus particulièrement qu'en utilisant dans une composition de dentifrice anhydre comprenant de la glycérine, au moins une hydroxyéthylcellulose à chaîne hydrophobe et au moins une silice pyrogénée dont la taille moyenne des particules primaires est inférieure à 40 nm, il était possible de préparer une composition de dentifrice présentant les qualités et les caractéristiques précitées et permettant de résoudre les problèmes posés par les dentifrices de l'état de la technique. Les compositions de dentifrice anhydre conformes à l'invention présentent l'avantage de permettre l'introduction d'actifs peu ou pas stables en milieu aqueux et ne nécessitent pas l'utilisation d'un agent conservateur. Ces compositions présentent par ailleurs les propriétés notamment rhéologiques requises pour un dentifrice c'est-à-dire une consistance suffisante à la tenue et permettant l'étalement du dentifrice sur la brosse à dents et un cassant permettant la coupure du boudin de dentifrice à la fin de l'étalement. Elles présentent par ailleurs de bonnes propriétés moussantes, de nettoyage, de polissage et d'abrasion.
Ces compositions présentent également une bonne homogénéité et sont particulièrement brillantes et facilement dispersibles en bouche.

L'invention a donc pour objet une composition de dentifrice anhydre à base de glycérine, d'hydroxyéthylcellose à chaîne hydrophobe, et de silice pyrogénée dont la taille moyenne des particules primaires est inférieure à 40 nm.

Un autre objet de l'invention est constitué par un procédé de nettoyage des dents mettant en oeuvre une telle composition.

D'autres objets de l'invention apparaîtront à la lecture de la description et des exemples qui suivent.

La composition de dentifrice conforme à l'invention est essentiellement caractérisée par le fait qu'elle contient dans un milieu anhydre contenant moins de 3% en poids d'eau et comprenant de la glycérine, au moins une hydroxyéthylcellulose comportant au moins une chaîne hydrophobe et au moins une silice pyrogénée dont la taille moyenne des particules primaires est inférieure à 40 nm.

Les hydroxyéthylcelluloses à chaîne hydrophobe plus particulièrement utilisables conformément à l'invention sont des hydroxyéthylcelluloses qui ont été modifiées par l'introduction d'un groupement alkyle ou aralkyle hydrophobe. Les hydroxyéthylcelluloses ont généralement un poids moléculaire compris entre 1000 et 1 000 000 et de préférence entre 50 000 et 500 000. Elles ont un degré de substitution moyen en groupements hydroxyéthyles compris entre 2 et 4 par unité anhydroglucose cellulosique de la molécule de cellulose.

La chaîne alkyle ou aralkyle hydrophobe peut être attachée au substrat éther cellulosique par l'intermédiaire d'une liaison éther, ester ou uréthane, les liaisons éthers étant préférées.

Le taux de substitution en groupements hydrophobes varie entre 0,2 % en poids par rapport au poids de l'éther cellulosique et la valeur qui entraîne une solubilité dans l'eau de l'éther cellulosique inférieure à 1 % en poids.

Le groupement alkyle a de préférence 10 à 24 atomes de carbone. Les groupements aralkyle désignent de préférence un groupement alkyl (C₆-C₂₀) phényl tel que nonylphényl, dodécylphényl.

Des produits de ce type et leur préparation sont décrits plus particulièrement dans les brevets US-A 4 228 277, EP-A 0 412 705 et EP-A -01 38 615.

De telles hydroxyéthylcelluloses à chaîne hydrophobe sont notamment commercialisées sous les dénominations de NATROSOL PLUS GRADE 330-CS et de POLYSURF 67 par la société AQUALON.

Le produit NATROSOL PLUS GRADE 330-CS est une hydroxyéthylcellulose substituée par 0,4 à 0,8 % en poids de groupes cétyles. Le degré de substitution molaire en hydroxyéthyl est compris entre 3 et 3,7 et la masse moléculaire avant modification chimique est d'environ 300 000.

Le POLYSURF 67 est substitué par 0,4 à 0,6 % de groupements cétyles par rapport au poids de l'hydroxyéthylcellulose, le degré de substitution molaire en groupements hydroxyéthyles étant compris entre 2,2 et 2,8.

Il est également possible d'utiliser conformément à l'invention des hydroxyéthylcelluloses comportant une chaîne hydrophobe aralkyle telles que décrites plus particulièrement dans le brevet EP-A 0384 167. A titre d'exemple, on peut citer plus particulièrement le produit commercialisé sous la dénomination AMERCELL POLYMER HM-1500 par la société AMERCHOL et qui est une hydroxéthylcellulose modifiée par un groupe nonoxynyl (nonylphénol).

On utilise de préférence les hydroxyéthylcelluloses à chaîne hydrophobe dans des concentrations comprises entre 0,01 et 2 % en poids et de préférence entre 0,05 et 0,5 % en poids par rapport au poids total de la composition.

Les silices pyrogénées utilisables conformément à l'invention ont une taille moyenne des particules primaires inférieure à 40 nm et de préférence inférieure ou égale à 30 nm, elles peuvent être de nature hydrophile ou hydrophobe. La taille moyenne des particules primaires est de préférence comprise entre 5 et 30 nm.

Parmi les silices pyrogénées hydrophiles dont la taille moyenne des particules primaires est inférieure à 40 nm, on peut citer les produits cornmercialisés sous les dénominations AEROSIL 90, AEROSIL 130, AEROSIL 150, AEROSIL 200, AEROSIL 300, AEROSIL 380 par la société DEGUSSA ainsi que le produit commercialisé sous la dénomination CAB-0-SIL-M-5 par la société CABOT.

Parmi les silices pyrogénées hydrophobes dont la taille moyenne des particules primaires est inférieure à 40 nm on peut citer les produits commercialisés sous les dénominations AEROSIL R202 AEROSIL R805, AEROSIL R812, AEROSIL R972, AEROSIL R974 par la société DEGUSSA, ainsi que le produit commercialisé sous la dénomination CAB-O-SIL-TS 720 par la société CABOT.

On utilise de préférence conformément à l'invention des silices pyrogénées de type hydrophobe.

Les silices pyrogénées sont présentes dans les compositions conformes à l'invention à une concentration comprise entre 2 et 10 % en poids de préférence entre 4 et 8 % en poids par rapport au poids total de la composition.

La glycérine utilisable selon l'invention est une glycérine pure telle que de préférence la glycérine pure CODEX à 99,8 %.

La glycérine représente plus de 50 % en poids par rapport au poids total de la composition et de préférence 65 à 90 % en poids.

Les compositions dentifrices conformes à l'invention contiennent généralement un agent de polissage dans des proportions pouvant varier entre 2 et 50 % en poids et de préférence 4 à 30 % en poids par rapport au poids total de la composition. Ces agents de polissage sont choisis plus particulièrement parmi les abrasifs minéraux constitués d'un ou plusieurs composés, généralement insolubles dans l'eau et dans la glycérine. On peut citer par exemple les métaphosphates de sodium ou de potassium, le phosphate de calcium dihydraté, le phosphate dicalcique, le phosphate tricalcique, le pyrophosphate de calcium, l'alumine, les alumines hydratées et en particulier trihydratées, les silices différentes des silices pyrogènes utilisées selon l'invention, les silicates d'aluminium ou de zirconium, la bentonite ainsi que l'orthophosphate de magnésium ou le phosphate trimagnésien.

Les compositions conformes à l'invention peuvent également contenir un ou plusieurs agents tensio-actifs suffisamment stables et moussants. Ces agents tensio-actifs peuvent être de nature anionique, amphotère, zwittérionique, cationique ou non-ionique, et on utilise de préférence des agents tensio-actifs anioniques et/ou non-ioniques.

De façon générale, les agents tensio-actifs sont présents dans des proportions variant entre 0.1 et 5 % et de préférence entre 0,5 et 3 % en poids par rapport au poids total de la composition.

Les compositions conformes à l'invention peuvent également contenir des agents actifs pour l'hygiène buccale et notamment des agents connus pour détruire la mauvaise haleine, tels que par exemple les cyclodextrines ou des composés du zinc qui sont des sels minéraux ou organiques tels que par exemple les halogénures de zinc, l'acétate de zinc, le citrate de zinc ou le fluorure de zinc ou des dérivés cétoniques tel que décrits dans le brevet français FR-A-2 678 828.

Les compositions conformes à l'invention peuvent également renfermer d'autres additifs usuels dans le domaine des compositions buccales tels que des agents antibiotiques, des agent édulcorants, humectants ou rafraîchissants, des agents conservateurs, des agents colorants, des arômes, des substances et agents aromatisants ou de sapidité, des agents peptisants, des agents plastifiants, des agents antibactériens ou bactéricides, des vitamines, des agents anticaries, des agents antitartre, des agents antitache, des agents antiplaque, des cicatrisants, des vasomoteurs, des agents antisaignement, des agents actifs sur les gencives.

Les compositions de dentifrice anhydre conformes à l'invention permettent en particulier d'utiliser des agents actifs pas ou peu stables en milieu aqueux tels que des enzymes, des agents libérateurs d'oxygène, des agents de blanchiment comme par les exemple les peroxydes, les bicarbonates et les perborates, des agents anticaries comme le fluorure d'étain, des vitamines et en particulier la vitamine C qui est un agent essentiel lors de la synthèse du collagène au niveau de la gencive.

Parmi les agents édulcorants utilisables, on peut citer le saccharose, le lactose, le fructose, le xylitol, le cyclamate de sodium, le maltose, le saccharinate de sodium, des mélanges d'α-glucosyl/stéviolglucoside, le D-Mannitol, l'Aspartame, l'Acesulfam K et leurs mélanges. Ces agents édulcorants peuvent être présents dans des concentrations allant jusqu'à 2 % en poids par rapport au poids total de la composition.

Comme agents humectants on peut citer le sorbitol anhydre, la di-et tri-glycérine, le xylitol, les polyols tels que par exemple le polyéthylèneglycol, le polypropylèneglycol et le propylèneglycol. Ces agents humectants peuvent être présents dans des proportions comprises entre 0,1 et 5 % en poids.

Parmi les agents rafraîchissants, on peut citer le menthol et l'éthylmaltol.

Bien que les agents conservateurs ne soient pas absolument indispensables dans les compositions conformes à l'invention ceux-ci peuvent néanmoins être utilisés pour assurer une bonne pureté bactériologique des compositions. Ils sont choisis plus particulièrement parmi le parahydroxybenzoate de méthyle, le parahydroxybenzoate de propyle, le benzoate de sodium et d'autres conservateurs usuels. Les concentrations sont généralement inférieures à 0,5 % en poids par rapport au poids total de la composition. Comme agents aromatisants, on peut utiliser les essences de menthe, d'anis, d'eucalyptus, de cannelle, de girofle, de sauge, de réglisse, ou de fruits comme le citron, l'orange, la mandarine ou la fraise.

On peut également utiliser à ce titre le salicylate de méthyle. Les substances aromatisantes lorsqu'elles sont utilisées peuvent l'être jusqu'à des proportions de 5 % en poids par rapport au poids total de la composition.

Les agents antibactériens sont des agents habituellement utilisés dans ce type de composition à usage buccal, on choisit de préférence des actifs dont certains sont des huiles essentielles ou des substances telles que la chlorhexidine ou ses sels comme le digluconate, le dichlorhydrate ou le diacétate de chlorhexidine, l'alexidine l'octénidine et leurs sels, l'hexétidine, le phénoxyéthanol, l'alcool phénéthylique et le triclosan. Les agents anti-bactériens sont généralement présents dans des proportions allant jusqu'à 10 % en poids par rapport au poids total de la composition et de préférence dans des proportions comprises entre 0,05 et 2 % en poids.

Il est également possible d'incorporer dans les compositions conformes à l'invention, des agents anticaries comme le monofluorophosphate de sodium, des fluorures de sodium et d'étain, des fluorures d'amines, des fluorures de polymère cationique tels que ceux décrits plus particulièrement. dans le brevet français FR-A 2 647 012.

Les exemples qui suivent sont destinés à illustrer l'invention sans pour autant présenter un caractère limitatif.

### EXEMPLE 1

On prépare une pâte dentifrice de composition suivante :

| | |
|---|---|
| - Silice pyrogénée hydrophobe dont la taille moyenne des particules primaires est de 16 nm vendue sous la dénomination AEROSIL R972 par DEGUSSA | 6 g |
| - Hydroxyéthylcellulose modifiée par une chaîne cétyle vendue sous la dénomination NATROSOL PLUS GRADE 330-CS par AQUALON | 0,1 g |
| - Silice hydratée vendue sous la dénomination TIXOSIL 73 par RHONE POULENC | 10 g |
| - Laurylsulfate de sodium | 1,5 g |
| - Parahydroxybenzoate de méthyle | 0,2 g |
| - Saccharinate de sodium | 0,1 g |
| - Dioxyde de titane | 1 g |
| - Arômes | 0,8 g |
| - Glycérine pure Codex qsp | 100 g |

### EXEMPLE 2

On prépare une pâte dentifrice de composition suivante :

Cette composition est identique à celle décrite dans l'exemple 1 sauf qu'elle contient 6 g de silice pyrogénée hydrophile dont la taille moyenne des particules primaires est de 12 nm vendue sous la dénomination AEROSIL 200 par DEGUSSA à la place de la silice pyrogénée AEROSIL R972.

### EXEMPLE 3

On prépare une pâte dentifrice de composition suivante :

Elle est identique à celle de l'exemple 1 sauf qu'elle contient 0,1 g d'hydroxyéthylcellulose modifiée par un groupe nonoxynyl vendue sous la dénomination AMERCELL POLYMER HM-1500 par AMERCHOL.

### EXEMPLE 4

On prépare la composition suivante :

| | |
|---|---|
| - Silice pyrogénée hydrophobe dont la taille moyenne des particules primaires est de 16 nm vendue sous la dénomination AEROSIL R972 par DEGUSSA | 4 g |
| - Hydroxyéthylcellulose modifiée par une chaîne cétyle vendue sous la dénomination NATROSOL PLUS GRADE 330-CS par AQUALON | 0,2 g |
| - Silice hydratée vendue sous la dénomination TIXOSIL 73 par RHONE POULENC | 8 g |
| - Laurylsulfate de sodium | 1,4 g |
| - Dioxyde de titane | 0,8 g |
| - Saccharinate de sodium | 0,08 g |
| - Arômes | 0,6 g |
| - Glycérine pure Codex qsp | 100,0 g |

### EXEMPLE 5

On prépare une pâte dentifrice de composition suivante :

| | |
|---|---|
| - Silice pyrogénée hydrophobe dont la taille moyenne des particules primaires est de 16 nm vendue sous la dénomination AEROSIL R972 par DEGUSSA | 2 g |
| - Hydroxyéthylcellulose modifiée par une chaîne cétyle vendue sous la dénomination NATROSOL PLUS GRADE 330-CS par AQUALON | 0,5 g |
| - Silice hydratée vendue sous la dénomination TIXOSIL 73 par RHONE POULENC | 12 g |
| - Laurylsulfate de sodium | 1 g |
| - Dioxyde de titane | 0,6 g |
| - Parahydroxybenzoate de méthyle | 0,2 g |
| - Saccharinate de sodium | 0,1 g |
| - Arômes | 0,8 g |
| - Glycérine pure Codex qsp | 100 g |

### EXEMPLE 6

On prépare une pâte dentifrice de composition suivante :

| | |
|---|---|
| - Silice pyrogénée hydrophobe dont la taille moyenne des particules primaires est de 16 nm vendue sous la dénomination AEROSIL R972 par DEGUSSA | 8 g |
| - Hydroxyéthylcellulose modifiée par une chaîne cétyle vendue sous la dénomination NATROSOL PLUS GRADE 330-CS par AQUALON | 0,05 g |
| - Silice hydratée vendue sous la dénomination TIXOSIL 73 par RHONE POULENC | 4 g |
| - Laurylsulfate de sodium | 1,8 g |
| - Dioxyde de titane | 1,0 g |
| - Parahydroxybenzoate de méthyle | 0,1 g |
| - Saccharinate de sodium | 0,08 g |
| - Arômes | 0,8 g |
| - Glycérine Codex qsp | 100 g |

### EXEMPLE 7

On prépare une pâte dentifrice ayant la même composition que celle de l'exemple 1 en utilisant comme silice pyrogénée hydrophile l'AEROSIL 90 (taille moyenne des particules primaires : 20 nm) de chez DEGUSSA : 6 g.

## Revendications

1. Composition de dentifrice caractérisée par le fait qu'elle contient dans un milieu anhydre contenant moins de 3% en poids d'eau et comprenant de la glycérine, au moins une hydroxyéthylcellulose comportant au moins une chaîne hydrophobe et au moins une silice pyrogénée dont la taille moyenne des particules primaires est inférieure à 40 nm.

2. Composition de dentifrice selon la revendication 1, caractérisée par le fait que l'hydroxyéthylcellulose à chaîne hydrophobe est choisie parmi les hydroxyéthylcelluloses modifiées par l'introduction d'un groupement alkyle ou aralkyle hydrophobe.

3. Composition de dentifrice selon la revendication 1 ou 2, caractérisée par le fait que les hydroxyéthylcelluloses ont un poids moléculaire compris entre 1000 et 1 000 000 et ont un degré de substitution moyen en groupements hydroxyéthyles compris entre 2 et 4 par unité anhydroglucose cellulosique de la molécule de cellulose.

4. Composition selon l'une quelconque des revendications 1 à 3, caractérisée par le fait que le taux de substitution en groupements hydrophobes varie entre 0,2 % en poids par rapport au poids de l'éther cellulosique et la valeur qui entraîne une solubilité dans l'eau de l'éther cellulosique inférieure à 1 % en poids.

5. Composition selon l'une quelconque des revendications 1 à 4, caractérisée par le fait que le groupement hydrophobe est choisi parmi les groupements alkyles ayant 10 à 24 atomes de carbone et des groupements aralkyles.

6. Composition selon l'une quelconque des revendications 1 à 5, caractérisée par le fait que l'on utilise des silices pyrogénées hydrophiles ou hydrophobes dont la taille moyenne des particules primaires est comprise entre 5 et 30 nm.

7. Composition selon l'une quelconque des revendications 1 à 6, caractérisée par la fait que la glycérine représente plus de 50 % en poids par rapport au poids total de la composition.

8. Composition selon l'une quelconque des revendications 1 à 7, caractérisée par le fait que les hydroxyéthylcelluloses à chaîne hydrophobe sont utilisées dans des proportions comprises entre 0,01 et 2 % en poids par rapport au poids total de la composition.

9. Composition selon l'une quelconque des revendications 1 à 8 caractérisée par le fait que les silices pyrogénées sont utilisées dans des proportions comprises entre 2 et 10 % en poids par rapport au poids total de la composition.

10. Composition de dentifrice selon l'une quelconque de revendications 1 à 9, caractérisée par le fait qu'elle renferme également un agent de polissage dans des proportions comprises entre 2 et 50 % en poids par rapport au poids total de la composition.

11. Composition selon l'une quelconque des revendications 1 à 10, caractérisée par le fait qu'elle renferme un ou plusieurs agents tensio-actifs moussants.

12. Composition selon l'une quelconque des revendications 1 à 11, caractérisée par le fait qu'elle contient également des agents actifs pour l'hygiène buccale.

13. Composition selon l'une quelconque des revendications 1 à 12, caractérisée par le fait qu'elle renferme au moins un additif choisi parmi les agents antibiotiques, les agents édulcorants, humectants ou rafraîchissants, les agents conservateurs, les agents colorants, les arômes, les substances et agents aromatisants ou de sapidité, les agents peptisants, les agents plastifiants, les agents antibactériens ou bactéricides, les vitamines, les agents anticaries, les agents antitartre, les agents antiplaque, les agents antitache, les cicatrisants, les vasomoteurs, les agents antisaignement, les agents actifs pour les gencives.

14. Composition selon l'une quelconque des revendications 1 à 13, caractérisée par le fait qu'elle peut renfermer des enzymes, et/ou des agents libérateurs d'oxygène, et/ou des agents de blanchiment.

15. Composition selon l'une quelconque des revendications 1 à 14, caractérisée par le fait qu'elle renferme des agents conservateurs destinés à préserver une bonie pureté bactériologique des compositions.

## Claims

1. Dentifrice composition, characterized in that it contains, in an anhydrous medium containing less than 3% by weight of water and comprising glycerol, at least one hydroxyethylcellulose containing at least one hydrophobic chain and at least one pyrogenic silica for which the mean size of the primary particles is less than 40 nm.

2. Dentifrice composition according to Claim 1, characterized in that the hydroxyethylcellulose containing a hydrophobic chain is chosen from hydroxyethylcelluloses modified by the introduction of a hydrophobic alkyl or aralkyl group.

3. Dentifrice composition according to Claim 1 or 2, characterized in that the hydroxyethylcelluloses have a molecular weight of between 1000 and 1 000 000 and have a mean degree of substitution with hydroxyethyl groups of between 2 and 4 per cellulose anhydroglucose unit of the cellulose molecule.

4. Composition according to any one of Claims 1 to 3, characterized in that the degree of substitution with hydrophobic groups varies between 0.2% by weight with respect to the weight of the cellulose ether and the value which results in a solubility in water of the cellulose ether of less than 1% by weight.

5. Composition according to any one of Claims 1 to 4, characterized in that the hydrophobic group is chosen from alkyl groups having 10 to 24 carbon atoms and aralkyl groups.

6. Composition according to any one of Claims 1 to 5, characterized in that use is made of hydrophilic or hydrophobic pyrogenic silicas for which the mean size of the primary particles is between 5 and 30 nm.

7. Composition according to any one of Claims 1 to 6, characterized in that the glycerol represents more than 50% by weight with respect to the total weight of the composition.

8. Composition according to any one of Claims 1 to 7, characterized in that the hydroxyethylcelluloses containing a hydrophobic chain are used in proportions of between approximately 0.01 and 2% by weight with respect to the total weight of the composition.

9. Composition according to any one of Claims 1 to 8, characterized in that the pyrogenic silicas are used in proportions of between 2 and 10% by weight with respect to the total weight of the composition.

10. Dentifrice composition according to any one of Claims 1 to 9, characterized in that it also contains a polishing agent in proportions of between approximately 2 and 50% by weight with respect to the total weight of the composition.

11. Composition according to any one of Claims 1 to 10, characterized in that it contains one or more foaming surface-active agents.

12. Composition according to any one of Claims 1 to 11, characterized in that it also contains active agents for oral hygiene.

13. Composition according to any one of Claims 1 to 12, characterized in that it contains at least one additive chosen from antibiotics, sweeteners, humectants, cooling agents, preservatives, dyes, fragrances, aromatizing or flavouring substances and agents, peptizing agents, plasticizers, antibacterials or bactericides, vitamins, agents for combating caries, tartar, plaque or stains, healing substances, vasomotor agents, agents for combating bleeding or active agents for the gums.

14. Composition according to any one of Claims 1 to 13, characterized in that it can contain enzymes and/or oxygen-releasing agents and/or whitening agents.

15. Composition according to any one of Claims 1 to 14, characterized in that it contains preservatives intended to retain a satisfactory bacteriological purity of the compositions.

## Patentansprüche

1. Zahnpastenzusammensetzung,
dadurch **gekennzeichnet**, daß
sie in einem wasserfreien Milieu, das weniger als 3 Gew.% Wasser enthält, Glycerin, mindestens eine Hydroxyethylcellulose, die mindestens eine hydrophobe Kette aufweist, und mindestens eine pyrogene Kieselsäure enthält, deren mittlere Primärpartikelgröße weniger als 40 nm beträgt.

2. Zahnpastenzusammensetzung gemäß Anspruch 1,
dadurch **gekennzeichnet**, daß
die Hydroxyethylcellulose mit hydrophober Kette aus Hydroxyethylcellulosen ausgewählt ist, die durch Einführung einer hydrophoben Alkyl- oder Aralkylgruppe modifiziert sind.

3. Zahnpastenzusammensetzung gemäß Anspruch 1 oder 2,
dadurch **gekennzeichnet**, daß
die Hydroxyethylcellulosen ein Molekulargewicht von 1000 bis 1 000 000 und einen mittleren Substitutionsgrad an Hydroxyethylgruppen von 2 bis 4 pro cellulosischer Anhydroglucose-Einheit des Cellulosemoleküls aufweisen.

4. Zusammensetzung gemäß einem der Ansprüche 1 bis 3,
dadurch **gekennzeichnet**, daß
der Substitutionsgehlt an hydrophoben Gruppierungen einen Wertebereich von 0,2 Gew.%, bezogen auf das Gewicht des Celluloseethers, bis zu dem Wert aufweist, der eine Wasserlöslichkeit des Celluloseethers von weniger als 1 Gew.% ergibt.

5. Zusammensetzung gemäß einem der Ansprüche 1 bis 4,
dadurch **gekennzeichnet**, daß
die hydrophobe Gruppierung aus Alkylgruppen mit 10 bis 24 Kohlenstoffatomen und aus Aralkylgruppen ausgewählt ist.

6. Zusammensetzung gemäß einem der Ansprüche 1 bis 5,
dadurch **gekennzeichnet**, daß
sie pyrogene hydrophile oder hydrophobe Kieselsäuren enthalten, deren mittlere Primärpartikelgröße 5 bis 30 nm beträgt.

7. Zusammensetzung gemäß einem der Ansprüche 1 bis 6,
dadurch **gekennzeichnet**, daß
das Glycerin mehr als 50 Gew.% ausmacht, bezogen auf das Gesamtgewicht der Zusammensetzung.

8. Zusammensetzung gemäß einem der Ansprüche 1 bis 7,
dadurch **gekennzeichnet**, daß
die Hydroxyethylcellulosen mit hydrophober Kette in Mengenanteilen von 0,01 bis 2 Gew.% enthalten sind, bezogen auf das Gesamtgewicht der Zusammensetzung.

9. Zusammensetzung gemäß einem der Ansprüche 1 bis 8,
dadurch **gekennzeichnet**, daß
die pyrogenen Kieselsäuren in Mengenanteilen von 2 bis 10 Gew.% enthalten sind, bezogen auf das Gesamtgewicht der Zusammensetzung.

10. Zahnpastenzusammensetzung gemäß einem der Ansprüche 1 bis 9,
dadurch **gekennzeichnet**, daß
sie auch ein Poliermittel in Mengenanteilen von 2 bis 50 Gew.% enthält, bezogen auf das Gesamtgewicht der Zusammensetzung.

11. Zusammensetzung gemäß einem der Ansprüche 1 bis 10,
dadurch **gekennzeichnet**, daß
sie ein oder mehrere schäumende oberflächenaktive Mittel enthält.

12. Zusammensetzung gemäß einem der Ansprüche 1 bis 11,
dadurch **gekennzeichnet**, daß
sie auch Wirkmittel für die Mundhygiene enthält.

13. Zusammensetzung gemäß einem der Ansprüche 1 bis 12,
dadurch **gekennzeichnet**, daß
sie mindestens ein Additiv enthält, ausgewählt aus Antibiotika, Süßungsmitteln, Feuchtigkeits- und Erfrischungsmitteln, Konservierungsmitteln, Färbemitteln, Aromastoffen, Geruchs- oder Geschmackssubstanzen und -mitteln, Peptisiermitteln, Plastifiziermitteln, antibakteriellen oder bakteriziden Mitteln, Vitaminen, Antikariesmitteln, Antizahnsteinmitteln, Mitteln gegen Zahnbelag, Antifleckenmitteln, Vernarbungsmitteln, Vasomotoren, blutstillenden Mitteln und aus Wirkmitteln für das Zahnfleisch.

14. Zusammensetzung gemäß einem der Ansprüche 1 bis 13,
dadurch **gekennzeichnet**, daß
sie Enzyme und/oder Sauerstoff-Freisetzungsmittel und/oder Bleichmittel enthält.

15. Zusammensetzung gemäß einem der Ansprüche 1 bis 14,
dadurch **gekennzeichnet**, daß
sie Konservierungsmittel zur Aufrechterhaltung einer guten bakteriologischen Reinheit der Zusammensetzungen enthält.
